# EUROPÄISCHE PATENTANMELDUNG

(11) **EP 2 149 341 A2**
(43) Veröffentlichungstag der Anmeldung: **03.02.2010**
(21) Anmeldenummer: 09009747.8
(22) Anmeldetag: 28.07.2009
(51) Int. Cl.: A61B 17/70

(54) **Befestigungselement zur Befestigung eines Implantats an einer Lamina eines Wirbels**

(30) Priorität: 28.07.2008 DE 102008035093
(71) Anmelder: Brehm, Peter, 91085 Weisendorf (DE)
(72) Erfinder: Brehm, Peter, 91085 Weisendorf (DE); Gluch, Herbert, Dr. med., 83026 Rosenheim (DE)
(74) Vertreter: Grünecker, Kinkeldey, Stockmair & Schwanhäusser Anwaltssozietät

(57) **Zusammenfassung**

Ein Befestigungselement zum Befestigen eines Implantats zur Korrektur einer Wirbelsäulendeformität an einer Lamina eines Wirbels umfasst eine Platte mit einem Auflageabschnitt zur Auflage auf der Lamina. Die Platte weist an einer Seite einen gekrümmten Abschnitt zum Einhängen der Platte in die Lamina auf. Eine Längsrichtung der Platte verläuft von der Seite mit dem gekrümmten Abschnitt zu einer Seite, die dem gekrümmten Abschnitt gegenüberliegt. Eine Querrichtung der Platte verläuft senkrecht zur Längsrichtung. Die Platte kann in der Querrichtung eine Breite von 7 mm bis 13 mm aufweisen. Die Platte kann ferner zwei Vorsprünge umfassen, die sich an den gekrümmten Abschnitt anschließen. Jeder Vorsprung kann dabei einen vom gekrümmten Abschnitt der Platte abgewandten Teil aufweisen, der gegenüber dem Auflageabschnitt angeordnet ist.

## Beschreibung

Die vorliegende Erfindung bezieht sich auf Befestigungselemente zum Befestigen eines Implantats zur Korrektur einer Wirbelsäulendeformität an einer Lamina eines Wirbels, sowie auf ein Implantat zur Korrektur einer Wirbelsäulendeformität.

Im Lauf des Lebens verändert sich der Knochen eines Menschen. Mit zunehmendem Alter tritt ein Verlust an Knochensubstanz auf, der mit einer Verminderung der Knochendichte einhergeht. Bei manchen Patienten tritt eine Osteoporose auf. Dabei handelt es sich um einen pathologischen Knochenschwund, der den organischen und den Mineralanteil des Knochens gleichzeitig betrifft. Dadurch bedingt können die Knochen instabil werden, und es können Deformationen von Knochen auftreten. Die degenerative Instabilität und/oder Deformation der Knochen kann insbesondere die Wirbelsäule des Patienten betreffen, in der es zu osteoporotischen Wirbelfrakturen, sogenannten Sinterungsbrüchen, kommen kann.

Eine nicht behandelte Instabilität und/oder Deformation der Wirbelsäule kann im Lauf der Zeit zum Verlust der Gehfähigkeit und damit zur Pflegebedürftigkeit des Patienten führen. Konservative Behandlungsmethoden wie zum Beispiel ein Korsett, physikalische Therapie und/oder eine Schmerztherapie haben sich bei älteren Patienten als nur begrenzt erfolgreich erwiesen.

Es wurde deshalb vorgeschlagen, die degenerative Instabilität und/oder Deformität der Wirbelsäule durch eine Vertebroplastie oder eine Kyphoplastie zu behandeln. Bei der Vertebroplastie wird unter Lokalanästhesie eine Hohlnadel durch die Bogenwurzeln eines von osteoporotischen Frakturen betroffenen Wirbels eingebracht. Anschließend spritzt man Knochenzement in den Wirbelkörper ein, der unter Hitzeentwicklung in kurzer Zeit aushärtet. Die Kyphoplastie ist eine Variante der Vertebroplastie, bei der vor dem Einbringen von Knochenzement die Deformation des Wirbels durch Aufpumpen eines Ballons im Wirbelkörper beseitigt oder zumindest verringert wird.

Da die Vertebroplastie und die Kyphoplastie minimal invasive Verfahren sind, eignen sie sich gut für ältere Patienten. Degenerative Instabilitäten und/oder Deformitäten können mit ihnen allerdings nicht vollständig behoben werden.

Es ist bekannt, Deformationen der Wirbelsäule wie zum Beispiel eine Skoliose und/oder eine Kyphose durch Einsetzen vom Implantaten zu behandeln. Dabei werden Wirbel des Patienten zuerst durch Bewegen des Patienten in einer gewünschten Stellung ausgerichtet. Anschließend werden die Wirbel durch das Implantat fest miteinander verbunden und so in der gewünschten Stellung fixiert.

Die Befestigung des Implantats an den Wirbeln kann mit Hilfe von Pedikelschrauben oder Laminahaken erfolgen. Derartige Befestigungsmittel sind in David S. Bradford et. al, Moe's textboook of scoliosis and other spinal deformities" 3rd ed., W.B. Saunders Company, 1978, ISBN 0-7216-5533-5 und in S. Terry Canale (ed.), Campbell's Operative Orthopedics, Mosby, 2003, ISBN 0-323-01240-X beschrieben. Pedikelschrauben sind Knochenschrauben, die in die Pedikel der Wirbel des Patienten eingeschraubt werden. Zum Verbinden der Wirbel können zwischen den Pedikelschrauben Stäbe aus Metall befestigt werden. Laminahaken sind hakenförmige Bauteile, die in Lamina der Wirbel des Patienten eingehängt werden können. Zwischen den Laminahaken können Metallstäbe befestigt werden. Durch die Metallstäbe werden die Laminahaken gegen den Knochen gedrückt, wodurch ein Herausrutschen der Laminahaken aus den Laminae verhindert wird.

Weitere Befestigungssysteme nach dem Stand der Technik sind beispielsweise in den Druckschriften DE 699 32 444 T2 und WO 94/17746 beschrieben.

Die bekannten Implantate zur Behandlung von Deformationen eignen sich jedoch nur begrenzt für die Behandlung älterer Patienten. Besonders in Fällen einer schweren Form der Osteopenie mit einem T-Score von unter -1,5 der Normalabweichung des Knochenmineralgehalts (BMD), sowie bei allen Formen der Osteoporose, ermöglichen die zur Verfügung stehenden Implantate keine ausreichende Stabilisierung oder korrigierende Aufrichtung der Wirbelsäule.

Ein Nachteil bekannter Implantate ist, dass an den Kontaktflächen zwischen den Knochen und dem Implantat ein relativ großer Druck auftreten kann. Die Druckbelastung auf Knochen, bei denen bereits ein gewisser Verlust an Knochensubstanz aufgetreten ist, kann an der Kontaktfläche Mikrofrakturen verursachen, die im weiteren Verlauf zur Destabilisierung des Implantats führen können.

Ein weiterer Nachteil bekannter Implantate ist, dass die Implantate während interoperativer Manipulationen wie dem Verbinden der Laminahaken oder Pedikelschrauben mit dem Metallstab sowie bei der Korrektur der Deformität mit einem Distraktions- Kompressions- oder Derotationsmanöver eine gewisse Instabilität aufweisen können. Die kann zum Verrutschen des Implantats führen, was die Operation erschweren und ihre Dauer verlängern kann.

Noch ein weiterer Nachteil bekannter Implantate ist die Rigidität vorhandener Systeme, die keine oder eine sehr geringe Beweglichkeit des Knochens relativ zum Implantat erlaubt. Dadurch können relativ große mechanische Belastungen der Kontaktfläche zwischen dem Knochen und dem Implantat auftreten, die zur Überlastung der Kontaktfläche führen können. Derartige Komplikationen können insbesondere während des Heilungsprozesses kurz nach der Operation auftreten.

Ein weiterer Nachteil bekannter Implantate ist, dass zum Anbringen bekannter Laminahaken das gelbe Band (Ligamentum flavum), das sich zwischen jeweils zwei Wirbeln befindet und die Wirbelsäule stabilisiert, entfernt werden muss, was zu einer Verringerung der Stabilität der Wirbelsäule führen kann.

Es ist eine Aufgabe der vorliegenden Erfindung, ein Befestigungselement zum Befestigen eines Implantats zur Korrektur einer Wirbelsäulendeformität sowie ein Implantat bereitzustellen, das eine Verringerung der Druckbelastung an den Kontaktflächen zwischen dem Knochen und dem Implantat ermöglicht.

Es ist eine weitere Aufgabe der vorliegenden Erfindung, ein Befestigungselement zum Befestigen eines Implantats zur Korrektur einer Wirbelsäulendeformität sowie ein Implantat bereitzustellen, das eine primäre Stabilität aufweist und so eine Vereinfachung der intraoperativen Manipulationen ermöglicht.

Noch eine weitere Aufgabe der vorliegenden Erfindung ist, ein Befestigungselement zum Befestigen eines Implantats zur Korrektur einer Wirbelsäulendeformität sowie ein Implantat bereitzustellen, das eine gewisse Beweglichkeit des Implantats relativ zum Knochen ermöglicht.

Noch eine weitere Aufgabe der vorliegenden Erfindung ist, ein Befestigungselement zum Befestigen eines Implantats zur Korrektur einer Wirbelsäulendeformität sowie ein Implantat bereitzustellen, bei dem das Entfernen des gelben Bandes vermieden werden kann.

Ein Befestigungselement zum Befestigen eines Implantats zur Korrektur einer Wirbelsäulendeformität an einer Lamina eines Wirbels gemäß einem Aspekt der Erfindung umfasst eine Platte mit einem Auflageabschnitt zur Auflage auf der Lamina.

Die Platte weist an einer Seite einen gekrümmten Abschnitt zum Einhängen der Platte in die Lamina auf. Eine Längsrichtung der Platte verläuft von der Seite mit dem gekrümmten Abschnitt zu einer Seite der Platte, die der Seite mit dem gekrümmten Abschnitt gegenüberliegt. Eine Querrichtung der Platte verläuft senkrecht zu der Längsrichtung. Die Platte weist in der Querrichtung eine Breite von 7 mm bis 13 mm auf.

Durch die im Vergleich zu bekannten Laminahaken größere Breite des Implantats kann die Kontaktfläche zwischen dem Befestigungselement und dem Knochen, auf der der Auflageabschnitt aufliegt, vergrößert werden. Dadurch können mechanische Belastungen des Knochens im Bereich der Kontaktfläche verringert werden. Da die Platte, anders als beispielsweise ein Pedikelhaken, nicht starr mit dem Knochen verbunden wird, ist eine Beweglichkeit des Befestigungselements sowie des Implantats, das mit Hilfe des Befestigungselements angebracht wird, relativ zum Knochen in gewissem Umfang möglich. Dadurch kann ein semirigides System bereitgestellt werden, was ebenfalls eine Verringerung der Belastung der Kontaktfläche zwischen dem Knochen und dem Befestigungselement ermöglicht. Die Breite der Platte im Bereich von 7 bis 13 mm ermöglicht es ferner, den gekrümmten Abschnitt während der Operation zwischen der Basis des Dornfortsatzes und der medialen Kante des Gelenkfortsatzes einzuklemmen, wodurch eine primäre Stabilität des Befestigungselements während der Operation bereitgestellt werden kann.

Geeigneterweise weist das Befestigungselement zwei Längsseiten auf, die parallel zu der Längsrichtung der Platte sind.

Geeigneterweise weist das Befestigungselement zwei oder mehr, beispielsweise drei oder mehr, Vorsprünge auf, die sich an den gekrümmten Abschnitt anschließen. Jeder Vorsprung weist einen vom gekrümmten Abschnitt der Platte abgewandten Teil auf, der gegenüber dem Auflageabschnitt der Platte angeordnet ist.

Das Befestigungselement kann in solchen Ausführungsformen derart in die Lamina eingehängt werden, dass sich die Vorsprünge in das gelbe Band einklemmen. So kann das Befestigungselement auch ohne eine Durchtrennung des gelben Bandes an der Lamina angebracht werden. Dies ermöglicht einen besseren Schutz des Bewegungssegments, das durch den Wirbel, an dem das Befestigungselement angebracht wird und einem benachbarten Wirbel, der durch das gelbe Band mit diesem verbunden ist, gebildet wird. Die Vorsprünge können außerdem ein Abrutschen des Befestigungselements von der Kante der Lamina, in die der gekrümmte Abschnitt eingehängt wird, verhindern. Dadurch wird die Stabilität des Befestigungselements verbessert.

Ein Befestigungselement zum Befestigen eines Implantats zur Korrektur einer Wirbelsäulendeformität an einer Lamina eines Wirbels gemäß einem weiteren Aspekt der Erfindung umfasst eine Platte, die an einer Seite einen gekrümmten Abschnitt zum Einhängen der Platte in die Lamina aufweist. An den gekrümmten Abschnitt schließen sich zwei oder mehr, beispielsweise drei oder mehr Vorsprünge an. Jeder Vorsprung weist einen vom gekrümmten Abschnitt der Platte abgewandten Teil auf, der gegenüber einem Auflageabschnitt der Platte angeordnet ist. Der Auflageabschnitt befindet sich zwischen dem gekrümmten Abschnitt und einer Seite der Platte, die der Seite mit dem gekrümmten Abschnitt gegenüberliegt.

Durch die Plattenform des Befestigungselements kann eine relativ große Kontaktfläche zwischen dem Knochen des Patienten und dem Befestigungselement bereitgestellt werden, die eine Verringerung der mechanischen Druckbelastung der Kontaktfläche ermöglicht. Nach dem Einhängen des gekrümmten Abschnitts in die Lamina bleibt eine gewisse Beweglichkeit des Befestigungselements relativ zum Knochen erhalten, so dass ein semirigides System bereit gestellt wird. Dadurch können Überbelastungen der Kontaktfläche zwischen dem Implantat und dem Knochen, die bei einem rigiden System auftreten könnten, vermieden werden. Die Vorsprünge können dabei helfen, ein Abrutschen des Befestigungselements von der Lamina, in die es eingehängt wird, zu vermeiden. Außerdem können die Vorsprünge in das gelbe Band eingeklemmt werden.

Geeigneterweise weist der Auflageabschnitt zwei Längsseiten auf, die parallel zu einer Längsrichtung der Platte sind. Die Längsrichtung verläuft von der Seite mit dem gekrümmten Abschnitt zu der Seite der Platte, die der Seite mit dem gekrümmten Abschnitt gegenüberliegt.

Geeigneterweise haben die Längsseiten einen Abstand im Bereich von 7 mm bis 13 mm.

Geeigneterweise verläuft eine Krümmungsachse des gekrümmten Abschnitts der Platte schräg zu der Längsrichtung. Geeigneterweise schließt die Krümmungsachse mit der Längsrichtung einen Winkel im Bereich von 95 bis 115 Grad ein. Dadurch kann eine verbesserte Anpassung der Form des Befestigungselements an die Form der Lamina erzielt werden.

Geeigneterweise weist der gekrümmte Abschnitt einen Oberflächenabschnitt auf, welcher der dem gekrümmten Abschnitt gegenüberliegenden Seite der Platte zugewandt ist, wobei für jeden Punkt in dem Oberflächenabschnitt gilt, dass ein Hauptnormalschnitt des Oberflächenabschnitts in dem Punkt in einer Ebene liegt, deren Normale schräg zu der Längsrichtung ist. Der gekrümmte Abschnitt kann dabei eine zylindrische Krümmung aufweisen, wobei eine Krümmungsachse des gekrümmten Abschnitts der Platte schräg zu der Längsrichtung verläuft. Beispielsweise kann die Krümmungsachse mit der Längsrichtung einen Winkel im Bereich von 95 bis 115 Grad einschließen. Alternativ dazu kann der Oberflächenabschnitt eine antiklastische Fläche sein, wobei der Mittelpunkt des zu dem genannten Hauptnormalschnitts gehörigen Krümmungskreises auf der Seite des gekrümmten Abschnitts liegt, die der dem gekrümmten Abschnitt gegenüberliegenden Seite der Platte zugewandt ist.

Eine antiklastische Form des gekrümmten Abschnitts ermöglicht eine bessere Anpassung der Form des Befestigungselements an die Form der menschlichen Laminakante, so dass der gekrümmte Abschnitt auf einer größeren Fläche auf der Laminakante aufliegt. Dadurch kann die mechanische Belastung des Kochens verringert und der Halt des Befestigungselements am Knochen verbessert werden. Eine zylindrische Form des gekrümmten Abschnitts hat den Vorteil einer im Vergleich zu einer antiklastischen Form einfacheren Herstellung.

Der genannte Hauptnormalschnitt kann der Hauptnormalschnitt mit minimalem Krümmungsradius sein.

In Ausführungsformen, in denen der gekrümmte Abschnitt eine zylindrische Krümmung aufweist, kann die Normale zur Ebene des Hauptnormalschnitts parallel zur Zylinderachse sein.

Geeigneterweise weist der Auflageabschnitt an einer der beiden Längsseiten einen Fortsatz auf, der sich in einer zur Längsrichtung der Platte schrägen Richtung erstreckt. Der Fortsatz kann zur Abstützung des Befestigungselements am Dornfortsatz des Wirbels, in dessen Lamina das Befestigungselement eingehängt wird, verwendet werden. Dadurch kann die primäre Rotationsstabilität des Befestigungselements verbessert werden.

Geeigneterweise ist der Fortsatz verformbar ausgebildet. So kann der Fortsatz während der Operation an die individuelle Anatomie des Patienten angepasst werden, beispielsweise durch Biegen des Fortsatzes mit Hilfe einer Zange.

Geeigneterweise weist der Auflageabschnitt eine konvexe Auflagefläche auf, wobei der gekrümmte Abschnitt zu der Auflagefläche hin gekrümmt ist. Die Lamina menschlicher Wirbelknochen hat eine sattelförmige Oberfläche, in deren Mitte sich eine Mulde befindet. Durch die konvexe Auflagefläche kann erreicht werden, dass die Auflagefläche des Auflageabschnitts sowohl in der Nähe der Seite des Befestigungselements mit dem gekrümmten Abschnitt und der gegenüberliegenden Seite des Befestigungselements, als auch in der Mitte der Lamina auf der Oberfläche der Lamina aufliegt. Dadurch kann eine Vergrößerung der Kontaktfläche zwischen dem Auflageabschnitt und der Lamina erzielt werden, die eine Verringerung mechanischer Belastungen des Knochens ermöglicht.

Geeigneterweise weist die Auflagefläche mindestens einen Punkt maximalen Abstands zu einer Ebene auf. Die Ebene verläuft durch Bereiche der Auflagefläche neben dem gekrümmten Abschnitt und durch Bereiche der Auflagefläche auf der Seite der Platte, die der Seite mit dem gekrümmten Abschnitt gegenüberliegt. Der mindestens eine Punkt maximalen Abstands hat von der Ebene einen Abstand im Bereich von 1 bis 2 mm. Eine derartige Geometrie der Auflagefläche ist der Form der Lamina besonders gut angepasst.

Geeigneterweise hat zumindest ein Teil der Auflagefläche eine konische Form. Eine zumindest teilweise konische Form ermöglicht eine große Kontaktfläche zwischen dem Befestigungselement und der Lamina.

Geeigneterweise weist der Auflageabschnitt eine konvexe Deckfläche auf, wobei der gekrümmte Abschnitt von der Deckfläche weg gekrümmt ist.

Geeigneterweise weist die Deckfläche mindestens einen Punkt maximalen Abstands zu einer Ebene auf. Die Ebene verläuft durch Bereiche der Deckfläche neben dem gekrümmten Abschnitt und durch Bereiche der Deckfläche auf der Seite der Platte, die der Seite mit dem gekrümmten Abschnitt gegenüberliegt. Der mindestens eine Punkt maximalen Abstands hat von der Deckfläche einen Abstand im Bereich von 3 bis 5 mm.

Geeigneterweise hat zumindest ein Teil der Deckfläche eine konische Form.

Geeigneterweise umfasst das Befestigungselement ferner ein Haltelement. Das Haltelement weist einen gekrümmten Abschnitt zum Einhängen des Haltelements in die Lamina auf. Es ist derart an dem Befestigungselement anbringbar, dass der gekrümmte Abschnitt des Haltelements dem gekrümmten Abschnitt des Befestigungselements gegenüber angeordnet ist.

Nach dem Einhängen des gekrümmten Abschnitts des Befestigungselements in eine Kante der Lamina ist die zweite Kante, die der Kante, in die der gekrümmte Abschnitt eingehängt wird, gegenüberliegt, noch frei. Der gekrümmte Abschnitt des Halteelements kann in die zweite Kante eingehängt werden und anschließend mit dem Befestigungselement verbunden werden. Dadurch kann das Befestigungselement primär stabil mit der Lamina verbunden werden, wodurch ein Verrutschen des Befestigungselements im weiteren Verlauf der Operation vermieden werden kann. Die Primärstabilität des mit dem Halteelement verbundenen Befestigungselements kann dabei die einer Pedikelschraube erreichen.

Ein Implantat zur Korrektur einer Wirbelsäulendeformität gemäß der vorliegenden Erfindung umfasst ein erstes Befestigungselement mit einigen oder allen der oben beschriebenen Merkmale und ein zweites Befestigungselement mit einigen oder allen der oben beschriebenen Merkmale. Außerdem umfasst das Implantat einen Verbindungsstab und Mittel zum Befestigen des Verbindungsstabs an dem ersten Befestigungselement und dem zweiten Befestigungselement.

Geeigneterweise umfassen das erste und das zweite Befestigungselement jeweils zwei oder mehr, beispielsweise drei oder mehr Vorsprünge. Diese schließen sich an den gekrümmten Abschnitt des Befestigungselements an. Die Vorsprünge des zweiten Befestigungselements sind länger als die Vorsprünge des ersten Befestigungselements.

Beim Einsetzen des Implantats kann der gekrümmte Abschnitt des zweiten Befestigungselement in die caudale Kante der Lamina eines Wirbels, die den Füßen des Patienten zugewandt ist, eingehängt werden und der gekrümmte Abschnitt des ersten Befestigungselements kann in die craniale Kante der Lamina eines anderen Wirbels, die dem Kopf des Patienten zugewandt ist, eingehängt werden. Wegen der anatomischen Form des menschlichen Wirbelknochens liegt die craniale Kante näher am Rückenmarkskanal als die caudale Kante. Durch die kürzeren Vorsprünge des ersten Befestigungselements kann ein größerer Abstand zwischen den Vorsprüngen und dem Rückenmark des Patienten eingehalten werden, wodurch das Risiko von Verletzungen des Rückenmarks verringert werden kann. An der caudalen Kante steht mehr Platz zwischen der Lamina und dem Rückenmark zur Verfügung. Deshalb kann hier der erforderliche Sicherheitsabstand zum Rückenmark auch mit längeren Vorsprüngen eingehalten werden, die eine bessere Stabilität des Befestigungselements ermöglichen.

Geeigneterweise ist das zweite Befestigungselement breiter als das erste Befestigungselement.

Geeigneterweise weist das zweite Befestigungselement einen Fortsatz auf, der sich in einer zur Längsrichtung der Platte schrägen Richtung erstreckt. Dadurch kann das zweite Befestigungselement zum Verbessern einer Rotationsstabilität am Dornfortsatz des Wirbels, in dessen Lamina das zweite Befestigungselement eingehängt wird, abgestützt werden.

Im Folgenden werden Ausführungsformen der Erfindung mit Bezug auf die beigefügten Zeichnungen beschrieben. Es zeigen:
Fig. 1a eine schematische Zeichnung eines Befestigungselements gemäß einer Ausführungsform der Erfindung;
Fig. 1b eine schematische Ansicht einer Platte des in Fig. 1a dargestellten Befestigungselements von der im implantierten Zustand der Lamina zugewandten Seite aus gesehen;
Fig. 1c eine schematische Ansicht eines Haltelements des in Fig. 1a dargestellten Befestigungselements von der im implantierten Zustand der Lamina zugewandten Seite aus gesehen;
Fig. 1d eine schematische Seitenansicht des in Fig. 1a dargestellten Befestigungselements von der im implantierten Zustand dem Dornfortsatz zugewandten Seite aus gesehen;
Fig. 2a eine schematische Ansicht der Platte des in Fig. 1a bis 1d dargestellten Befestigungselements in geradegebogenem Zustand von der im implantierten Zustand der Lamina zugewandten Seite aus gesehen;
Fig. 2b eine schematische Ansicht der Platte des in Fig. 1a bis 1d dargestellten Befestigungselements in geradegebogenem Zustand von der im implantierten Zustand dem Dornfortsatz zugewandten Seite aus gesehen;
Fig. 3a bis 3d schematische Ansichten einer Wirbelsäule in Stadien einer Implantation eines Implantats gemäß der vorliegenden Erfindung;
Fig. 4 eine schematische Perspektivansicht einer Oberfläche einer Lamina;
Fig. 5a eine schematische Zeichnung eines Befestigungselements gemäß einer weiteren Ausführungsform der Erfindung;
Fig. 5b eine schematische Perspektivansicht eines gekrümmten Abschnitts des in Fig. 5a dargestellten Befestigungselements; und
Fig. 5c eine schematische Ansicht der Platte des in Fig. 5a und 5b dargestellten Befestigungselements in geradegebogenem Zustand von der im implantierten Zustand dem Dornfortsatz zugewandten Seite aus gesehen.

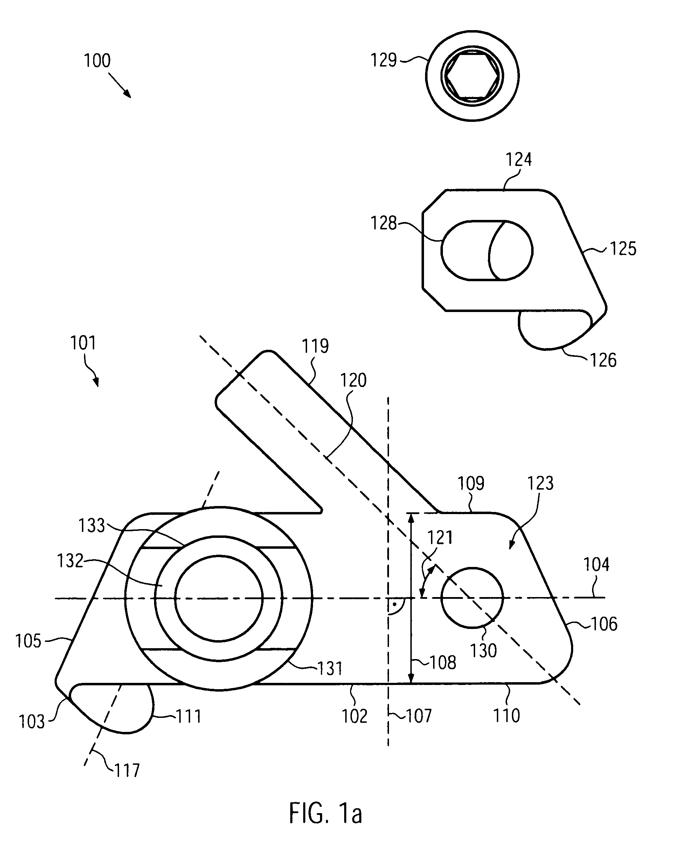

Fig. 1a zeigt eine schematische Zeichnung eines Befestigungselements 100 zum Befestigen eines Implantats zur Korrektur einer Wirbelsäulendeformität gemäß einer Ausführungsform der vorliegenden Erfindung.

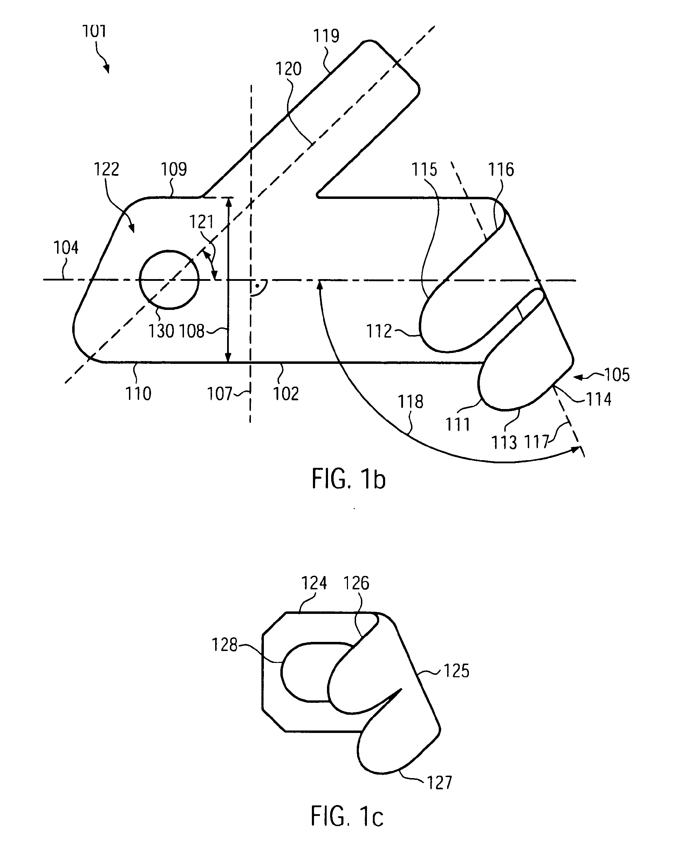

Das Befestigungselement 100 umfasst eine Platte 101. Die Platte 101 umfasst einen Auflageabschnitt 102. Wenn das Befestigungselement 100 einem Patienten implantiert wird, liegt der Auflageabschnitt 102 auf der Lamina eines Wirbels des Patienten auf, wobei eine Fig. 1a verdeckte Auflagefläche die Lamina berührt. In Fig. 1b ist eine schematische Ansicht der Platte 101 aus einer Blickrichtung gezeigt, in der die Auflagefläche, die durch das Bezugszeichen 122 bezeichnet ist, sichtbar ist.

Das Befestigungselement kann in manchen Ausführungsformen ein Halteelement 124 umfassen, das mit Hilfe eines Befestigungselements, beispielsweise einer Schraube 129, die durch Öffnungen 128, 130 im Haltelement 124 und in der Platte 101 geführt werden kann, an der Platte 101 angebracht werden kann. Zu diesem Zweck kann in der Öffnung 130 ein Gegengewinde zum Gewinde der Schraube 129 vorgesehen sein.

Eine schematische Ansicht des Halteelements 124 aus der Blickrichtung der Fig. 1b ist in Fig. 1c gezeigt. Fig. 1c zeigt eine schematische Seitenansicht des Befestigungselements 100.

An einer Seite 105 weist die Platte einen gekrümmten Abschnitt 103 auf. An den gekrümmten Abschnitt 103 schließen sich zwei Vorsprünge 111, 112 an. Der gekrümmte Abschnitt 103 ist zu der Auflagefläche 122 hin gekrümmt. Vom gekrümmten Abschnitt 103 abgewandte Teile 113, 115 der Vorsprünge 111, 112, die weiter vom gekrümmten Abschnitt 103 entfernt sind als Teile 114, 116, die dem gekrümmten Abschnitt 103 zugewandt sind, befinden sich deshalb gegenüber der Auflagefläche 122 des Auflageabschnitts 102.

Zusammen mit dem Auflageabschnitt 102 sowie den Vorsprüngen 111, 112 bildet der gekrümmte Abschnitt 103 somit eine hakenförmige Struktur, die, wie später genauer ausgeführt wird, in die Lamina eines Wirbels des Patienten eingehängt werden kann, so dass sich ein Teil der Lamina zwischen der Auflagefläche 122 und den Vorsprüngen 111, 112 befindet.

Die vorliegende Erfindung ist nicht auf Ausführungsformen beschränkt, in denen die Vorsprünge 111, 112 vorhanden sind. In anderen Ausführungsformen können die Vorsprünge 111, 112 auch weggelassen werden. In solchen Ausführungsformen wird eine hakenförmige Struktur zum Einhängen in die Lamina durch den gekrümmten Abschnitt 103 und den Auflageabschnitt 102 bereitgestellt. In manchen dieser Ausführungsformen kann sich an den gekrümmten Abschnitt 103 ein gerader Abschnitt anschließen, der gegenüber dem Auflageabschnitt 102 angeordnet ist, dort, wo sich in der Ausführungsform der Fig. 1a bis 1d die Vorsprünge 111, 112 befinden.

Die vorliegende Erfindung ist nicht auf Ausführungsformen beschränkt, in denen zwei Vorsprünge 111, 112 vorhanden sind. In anderen Ausführungsformen können auch drei oder mehr Vorsprünge mit Merkmalen entsprechend derer der Vorsprünge 111, 112 vorhanden sein.

Gegenüber der Auflagefläche 122 weist der Auflageabschnitt 102 eine Deckfläche 123 auf, die nach der Implantation des Befestigungselements 100 von der Lamina des Wirbels des Patienten abgewandt ist. Der gekrümmte Abschnitt 103 ist von der Deckfläche 123 weg gekrümmt.

Das Befestigungselement 100 umfasst ferner ein Verbindungselement 131, das derart an der Platte 101 angebracht ist, dass es sich vor der Deckfläche 123 und auf der der Auflagefläche 122 gegenüberliegenden Seite der Platte 101 befindet. Das Verbindungselement 131 ist dafür ausgebildet, das Befestigungselement 100 mit anderen Komponenten eines Implantats zur Korrektur einer Wirbelsäulendeformität wie beispielsweise einem Verbindungsstab zu verbinden.

Das Verbindungselement 131 kann ein Verbindungselement eines den Fachleuten bekannte Typs sein. In machen Ausführungsformen kann das Verbindungselement beispielsweise eine U-förmige Vertiefung 132 aufweisen, in die der Verbindungsstab eingesetzt werden kann, sowie ein Gewinde 133, in das eine Schraube zum Fixieren des Verbindungsstabs eingeschraubt werden kann.

In manchen Ausführungsformen kann das Verbindungselement 131 gelenkig mit der Platte 130 verbunden sein, um die Ausrichtung des Verbindungselements 130 zum Verbindungsstab zu verbessern, nachdem das Befestigungselement 100 mit dem Wirbelknochen des Patienten verbunden ist.

Eine Längsrichtung der Platte 101, die in den Fig. 1a und 1b durch eine gestrichelte Linie 104 dargestellt ist, verläuft von der Seite 105 der Platte 101, auf der sich der gekrümmte Abschnitt 103 befindet, zu einer Seite 106 der Patte 101, die der Seite 105 gegenüberliegt. Eine Querrichtung 107 der Platte 101 ist zu der Längsrichtung 104 senkrecht. In manchen Ausführungsformen kann die Platte 101 zwei Längsseiten 109, 110 haben, die zu der Längsrichtung 104 parallel sind. Die Seite 106 der Platte 101 kann in manchen Ausführungsformen parallel zu der Querrichtung 107 verlaufen. In anderen Ausführungsformen kann die Seite 106 schräg zu der Querrichtung 107 verlaufen. Dadurch kann die Form der Platte 101 exakter an die Form der Lamina menschlicher Wirbelknochen angepasst werden.

Eine Breite 108 der Platte 101 in der Querrichtung 107 ist in Fig. 1a und 1b durch das Bezugsreichen 108 bezeichnet. In Ausführungsformen, in denen die Längsseiten 109, 110 parallel zu der Längsrichtung 104 sind, entspricht die Breite 108 einem Abstand der beiden Längsseiten 109, 110. Die Breite 108 kann einer Breite der Lamina eines Wirbelknochens entsprechen. In manchen Ausführungsformen kann die Breite 108 einen Wert im Bereich von 7 bis 13 mm haben. In anderen Ausführungsformen kann die Breite 108 einen Wert von ungefähr 8 mm, ungefähr 10 mm oder ungefähr 12 mm haben.

In Ausführungsformen, in denen die Längsseiten 109, 110 nicht parallel zu einander sind, kann die Breite 108 in der Mitte zwischen dem gekrümmte Abschnitt 103 und der Seite 106 der Platte gemessen werden, wobei sie ebenfalls einen Wert im Bereich von 7 bis 13 mm haben kann.

Der gekrümmte Abschnitt 103 kann in manchen Ausführungsformen eine zylindrische Krümmung aufweisen, wobei eine Krümmungsachse 117 des gekrümmten Abschnitts 103 in manchen Ausführungsformen mit der Längsrichtung 104 einen Winkel 118 einschließt, der sich von einem rechten Winkel unterscheidet. In solchen Ausführungsformen verläuft die Krümmungsachse 117 somit schräg zu der Längsrichtung 104. Der Winkel 118 kann einen Wert im Bereich von 95 bis 105 Grad aufweisen, so dass die Krümmungsachse 117 einen Winkel zwischen 5 und 15 Grad mit der Querrichtung 107 einschließt. In einer Ausführungsform der Erfindung kann der Winkel 118 einen Wert von 100 Grad haben.

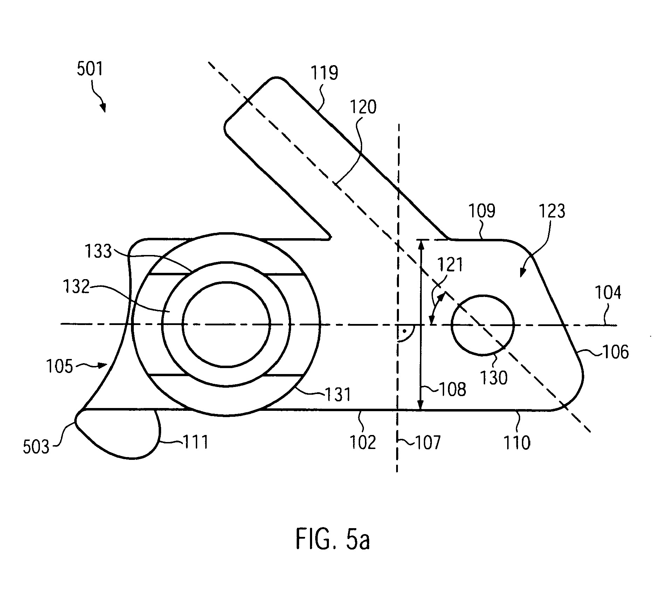

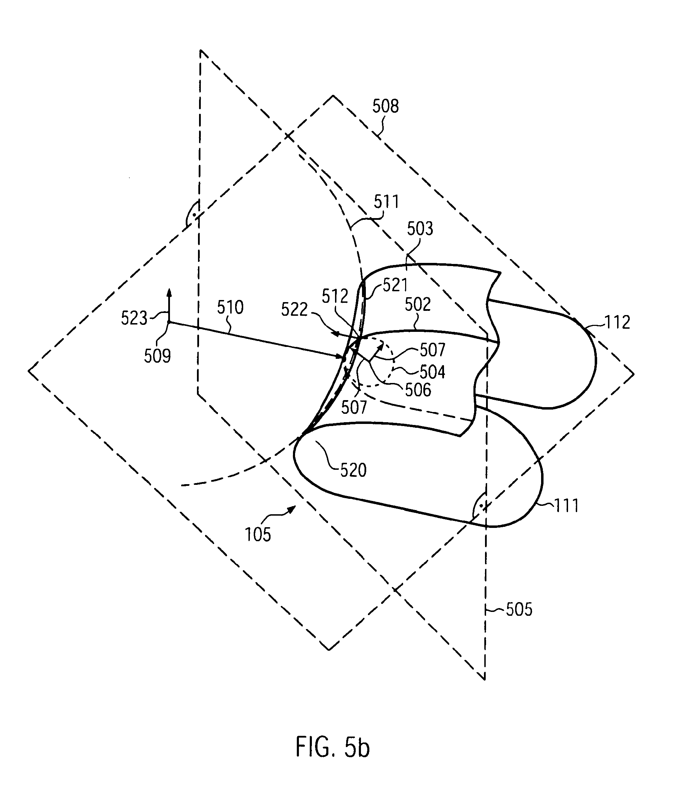

Die vorliegende Erfindung ist jedoch nicht auf Ausführungsformen beschränkt, in denen der gekrümmte Abschnitt eine zylindrische Krümmung aufweist. Fig. 5a zeigt eine schematische Ansicht einer Platte 501 einer anderen Ausführungsform, in der anstelle eines zylindrisch gekrümmten Abschnitts ein gekrümmter Abschnitt 503 mit einer antiklastischen Form vorgesehen ist. Die weiteren Merkmale der Platte 501 können denen der Platte 101 entsprechen. Eine Detailansicht des gekrümmten Abschnitts 503 ist in Fig. 5b gezeigt. Der Übersichtlichkeit halber wurden in Fig. 5b einige Teile der Platte 501 weggelassen.

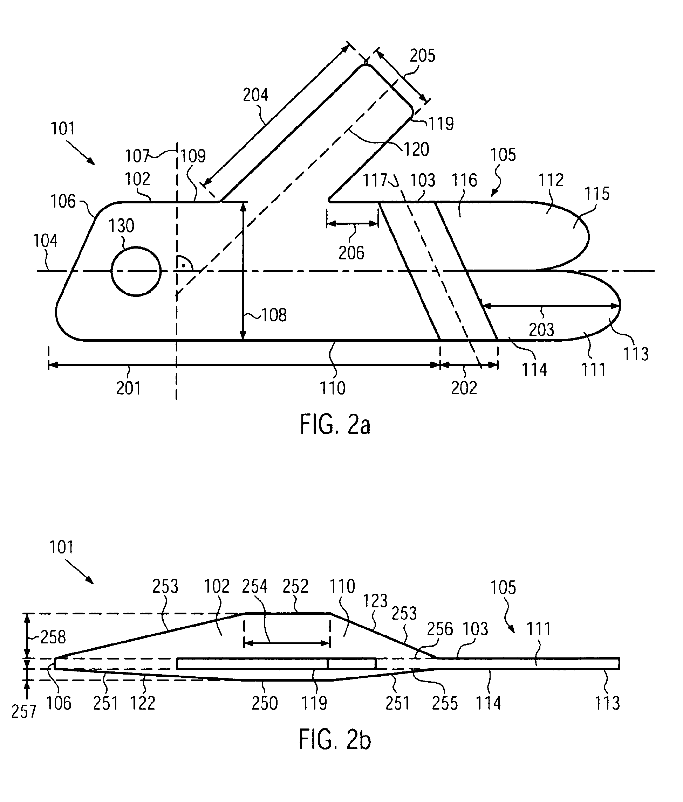

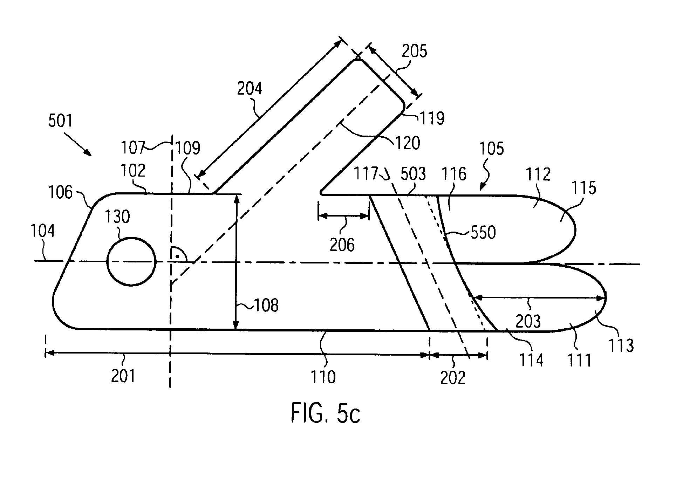

Der gekrümmte Abschnitt 503 weist einen Oberflächenabschnitt 520 auf. Der Oberflächenabschnitt 520 befindet sich auf einer Seite des gekrümmten Abschnitts 503, welche der vom gekrümmten Abschnitt 503 abgewandten Seite 106 der Platte 501 zugewandt ist. Der Oberflächenabschnitt 520 befindet sich somit, falls die Platte 501, wie in Fig. 2a, 2b und 5c dargestellt, geradegebogen wird, auf der gleichen Seite der Platte 501 wie die Auflagefläche 122. Somit kann der Oberflächenabschnitt 520 im implantierten Zustand der Platte den Wirbel des Patienten berühren, während die dem Oberflächenabschnitt 520 gegenüberliegende Seite des gekrümmten Abschnitts 503 vom Knochen abgewandt ist.

Der Oberflächenabschnitt 520 kann sich in manchen Ausführungsformen über die gesamte Breite 108 der Platte 501 in der Querrichtung 107 erstrecken. In anderen Ausführungsformen kann am Rand des gekrümmten Abschnitts 503 neben dem Oberflächenabschnitt 520 auch ein abgeschrägter oder abgerundeter Bereich vorgesehen sein, damit die Platte 501 weniger scharfkantig ist. In manchen dieser Ausführungsformen kann sich der Oberflächenbereich 520 über 90% oder mehr der Breite 108 erstrecken.

In Fig. 5b bezeichnet das Bezugszeichen 512 einen Punkt in dem Oberflächenabschnitt 520. Die Ebene eines ersten Hauptnormalschnitts 502 des Oberflächenabschnitts 520 im Punkt 512 ist durch das Bezugszeichen 505 bezeichnet. Das Bezugszeichen 508 bezeichnet die Ebene des zweiten Hauptnormalschnitts 521 im Punkt 520. Die Ebenen 505, 508 verlaufen durch den Punkt 512, stehen senkrecht aufeinander und sind parallel zu einer Oberflächennormalen 522 des Oberflächenabschnitts 520 im Punkt 512.

Der erste Hauptnormalschnitt 502 ist eine Schnittkurve des Oberflächenabschnitts 520 mit der Ebene 505. Ein Krümmungskreis des ersten Hauptnormalschnitts 502 im Punkt 520 mit Mittelpunkt 506 und Radius 507 ist durch das Bezugszeichen 504 bezeichnet. Der Krümmungskreis 504 liegt in der Ebene 505, berührt den ersten Hauptnormalschnitt 502 im Punkt 520 und hat eine Krümmung, die gleich der Krümmung des Hauptnormalschnitts 502 im Punkt 520 ist.

Entsprechend ist der zweite Hauptnormalschnitt 521 eine Schnittkurve des Oberflächenabschnitts 520 mit der Ebene 508. Ein Krümmungskreis 511 des zweiten Hauptnormalschnitts 521 im Punkt 512 liegt in der Ebene 505, berührt den zweiten Hauptnormalschnitt 511 im Punkt 512, hat einen Radius 510 und einen Mittelpunkt 509. Eine Krümmung des Krümmungskreises 511 ist gleich der Krümmung des zweiten Hauptnormalschnitts 521 im Punkt 512.

Die Orientierung der Ebenen 505, 508 ist, entsprechend der mathematischen Definition der Hauptnormalschnitte, so gewählt, dass der Krümmungsradius 507 ein Minimum der Krümmungsradien der Schnittlinien des Oberflächenabschnitts 520 mit allen Ebenen, die durch den Punkt 512 verlaufen und parallel zum Normalenvektor 522 sind, ist, und der Krümmungsradius 510 ein Maximum aller derartigen Krümmungsradien ist. Die Krümmungsradien der Schnittlinien sind dabei die Krümmungsradien im Punkt 512.

Aufgrund der antiklastischen Form des gekrümmten Abschnitts 503 liegen die Mittelpunkte 506, 509 auf verschiedenen Seiten des Oberflächenabschnitts 520. Der Mittelpunkt 506 liegt auf der Seite des gekrümmten Abschnitts 503, die der dem gekrümmten Abschnitt 503 gegenüberliegenden Seite 106 der Platte 501 zugewandt ist. Der Mittelpunkt 509 liegt auf der Seite des gekrümmten Abschnitts, die von der dem gekrümmten Abschnitt 503 gegenüberliegenden Seite 106 der Platte abgewandt ist. Der Oberflächenabschnitt 520 weist somit eine Sattelform auf, die der Form der Kante der Lamina des menschlichen Wirbelknochens entspricht. Dadurch kann eine bessere Anpassung der Form des gekrümmten Abschnitts 503 an die Form der Kante der Lamina eines menschlichen Wirbelknochens erreicht werden.

In manchen Ausführungsformen kann der Oberflächenabschnitt 520 zumindest näherungsweise die Form eines hyperbolischen Paraboloids haben. In anderen Ausführungsformen kann der Oberflächenabschnitt 520 zumindest näherungsweise die Form eines Rotationshyperboloids haben. Zwischen dem Oberflächenabschnitt 520 und der Auflagefläche 122 und/oder zwischen dem Oberflächenabschnitt 520 und den Vorsprüngen 111, 112 kann sich ein Übergangsbereich befinden, so dass sich ein stetiger Übergang zwischen der Form des Oberflächenabschnitts 520 und der Form der Auflagefläche 122 bzw. der Form der Vorsprünge 111, 112 ergibt.

In Fig. 5b bezeichnen die Bezugszeichen 507, 523 Normalenrichtungen der Ebenen 505 und 508. Die Normalenrichtung der Ebene 505 des ersten Hauptnormalschnitts 502 mit minimalem Krümmungsradius 507 im Punkt 512 kann dabei bei allen Punkten 512 im Oberflächenabschnitt 520 schräg zu der Längsrichtung 104 sein.

In manchen Ausführungsformen kann der mittlere Winkel der Normalen 507 zu der Ebene des ersten Hauptnormalschnitts 502, wobei die Mittelung über alle Punkte 512 im Oberflächenabschnitt 520 durchgeführt wird, einen Wert im Bereich von 95 bis 115 Grad, beispielsweise einen Wert von 100 Grad haben.

In manchen Ausführungsformen kann der Winkel zwischen der Normalen 507 zu der Ebene des ersten Hauptnormalschnitts 502 und der Längsrichtung 104 in einem, mehreren oder allen Punkten 512, die sich in der Mitte zwischen den Längsseiten 109, 110 befinden, einen Wert im Bereich von 95 bis 115 Grad, beispielsweise einen Wert von 100 Grad, haben.

In Ausführungsformen, in denen der Oberflächenabschnitt 520 eine rotationssymmetrische Form hat, beispielsweise die Form eines Rotationshyperboloids, kann die Rotationsachse mit der Längsrichtung 104 einen Winkel im Bereich von 95 bis 115 Grad, beispielsweise einen Winkel von 100 Grad, einschließen.

Die oben mit Bezug auf Fig. 5b beschriebene Definition des ersten Hauptschnitts mit minimalem Krümmungsradius und des zweiten Hauptschnitts mit maximalem Krümmungsradius, sowie der zugehörigen Ebenen, gilt auch für nicht antiklastische Formen des gekrümmten Abschnitts 503. Im Fall des mit Bezug auf Fig. 1a bis 1d beschriebenen gekrümmten Abschnitts mit zylindrischer Krümmung ist die Richtung der Normalen zu der Ebene des Hauptschnitts mit minimalen Krümmungsradius durch einen Punkt der zylindrischen Oberfläche des gekrümmten Abschnitts parallel zu der Richtung der Krümmungsachse 117. Im zweiten Hauptschnitt erhält man im Fall einer zylindrischen Oberfläche einen unendlich großen Krümmungsradius.

In manchen Ausführungsformen kann der gekrümmte Abschnitt 103, 503 um einen Winkel von ungefähr 180 Grad zu dem Auflageabschnitt 102 gebogen sein und die Vorsprünge 111, 112 können annähernd gerade sein, so dass sie in einer Ebene liegen, die annähernd parallel zu einer Ebene durch die Längsrichtung 104 und die Querrichtung 107 ist.

In anderen Ausführungsformen kann der gekrümmte Abschnitt 103, 503 um einen Winkel von weniger als 180 Grad zum Auflageabschnitt 102 gebogen sein, so dass die dem gekrümmten Abschnitt 103, 503 zugewandten Teile 114, 116 der Vorsprünge zu der Ebene durch die Längsrichtung 104 und die Querrichtung 107 geneigt sind. In manchen dieser Ausführungsformen können die Vorsprünge 111, 112 gerade sein, so dass die gesamten Vorsprünge 111, 112 zu der Ebene durch die Längsrichtung 104 und die Querrichtung 107 geneigt sind.

In anderen Ausführungsformen können die dem gekrümmten Abschnitt 103, 503 zugewandten Teile 114, 116 in der selben Richtung gekrümmt sein wie der gekrümmte Abschnitt 103 bzw, im Fall einer antiklastischen Form des gekrümmten Abschnitts 503, die antiklastische Form des gekrümmten Abschnitts 503 fortsetzen. In einer Ausführungsform ist der gekrümmte Abschnitt 103, 503 um einen Winkel von ungefähr 90 Grad zum Auflageabschnitt 102 hin gebogen und die dem gekrümmten Abschnitt 103 zugewandten Teile 114, 116 sind ebenfalls um einen Winkel von ungefähr 90 Grad zum Auflageabschnitt 102 hin gebogen, so dass die vom gekrümmten Abschnitt 103 abgewandten Teile 113, 115 annähernd parallel zu der Ebene durch die Längsrichtung 104 und die Querrichtung 107 sind.

In manchen Ausführungsformen kann der Auflageabschnitt 102 an einer der Längsseiten 109, 110, beispielsweise der Längsseite 109, einen Fortsatz 119 aufweisen. Der Fortsatz 119 kann sich entlang einer Richtung 120 erstrecken, die mit der Längsrichtung 104 einen Winkel einschließt, der in Fig. 1b durch das Bezugszeichen 121 bezeichnet ist. Der Winkel 121 kann einen Wert kleiner als 90 Grad aufweisen, so dass der Fortsatz 119 schräg zu der Längsrichtung 104 ist. Der Fortsatz 119 ist somit zu dem gekrümmten Abschnitt 103, 503 hin geneigt und von der Seite 106 der Platte 101, die dem gekrümmten Abschnitt 103, 503 gegenüberliegt, weggeneigt.

Wie oben ausgeführt, kann die Krümmungsachse 117 schräg zu der Längsrichtung 104 und der Querrichtung 107 verlaufen. In manchen Ausführungsformen der Erfindung kann die Krümmungsachse 117 zu dem Fortsatz 119 hin geneigt sein, wobei der Winkel 118 zwischen der Krümmungsachse 117 und der Längsrichtung 104, der sich auf der vom Fortsatz 119 abgelegenen Seite der Längsrichtung 104 befindet, wie oben beschrieben einen Wert größer als 90 Grad annimmt.

In manchen Ausführungsformen kann die dem gekrümmten Abschnitt 103 gegenüberliegende Seite 106 der Platte 101, 501 ebenfalls zu dem Fortsatz 119 hin geneigt sein, so dass der Auflageabschnitt 102 eine trapezförmige Gestalt hat. Die Längsseite 109, an der sich der Fortsatz 119 befindet, ist dabei kürzer als die dem Fortsatz 119 gegenüberliegende Längsseite 110.

In manchen Ausführungsformen kann der Winkel 121 einen Wert im Bereich von 30 bis 60 Grad, beispielsweise einen Wert von ungefähr 45 Grad haben.

Der Fortsatz 119 ist dafür ausgebildet, in den Dornfortsatz des Wirbels des Patienten, an dem das Befestigungselement 100 befestigt wird, eingehängt zu werden. In manchen Ausführungsformen ist der Fortsatz 119 verformbar. Während der Implantation kann der Fortsatz 119 mit einem Werkzeug, beispielsweise einer Zange, in eine hakenförmige Form gebogen werden, die der individuellen Anatomie des Patienten angepasst ist.

In manchen Ausführungsformen kann der Fortsatz 119 zunächst im wesentlichen gerade ausgebildet sein. In anderen Ausführungsformen kann der Fortsatz 119 bereits bei der Herstellung des Befestigungselements 100 hakenförmig ausgebildet werden.

Die vorliegende Erfindung ist nicht auf Ausführungsformen beschränkt, in denen der Auflageabschnitt 102 einen Fortsatz 119 aufweist. In anderen Ausführungsformen kann der Fortsatz 119 weggelassen werden. Auch in solchen Ausführungsformen kann der Auflageabschnitt 102 jedoch eine trapezförmige Gestalt haben, wobei die Krümmungsachse 117 und die diesem gegenüberliegende Seite 106 einen spitzen Winkel einschließen, so dass eine der beiden Längsseiten 109, 110, beispielsweise die Längsseite 109, kürzer ist als die andere.

Das Haltelement 124 kann einen gekrümmten Abschnitt 125 ähnlich dem gekrümmten Abschnitt 103, 503 der Platte 101, 501 sowie Vorsprünge 126, 127 ähnlich den Vorsprüngen 112, 113 aufweisen. Dadurch kann das Haltelement 124 in die Lamina des Wirbels, an dem das Befestigungselement 100 angebracht wird, eingehängt werden, und zwar an einer Kante der Lamina, die derjenigen Kante, an welcher der gekrümmte Abschnitt 103, 503 eingehängt wird, gegenüberliegt. Ein Teil der Lamina kann sich nach dem Einhängen zwischen den Vorsprüngen 126, 127 und einem Abschnitt des Haltelements, der auf der den Vorsprüngen 126, 127 gegenüberliegenden Seite des gekrümmten Abschnitts 125 angeordnet ist, und/oder zwischen den Vorsprüngen 126, 127 und einem Teil des Auflageabschnitts 102 in der Nähe der Seite 106 befinden.

Wenn das Haltelement 124 mit der Platte 101, 501 verbunden ist, z.B. durch Verschrauben mit Hilfe der Schraube 129, kann das Befestigungselement durch die oben beschriebene Form der Platte 101, 501 und des Haltelements 124 eine klammerförmige Gestalt erhalten, die dafür geeignet ist, die Lamina eines Wirbels zu umschließen. Dadurch kann eine primär stabile Befestigung des Befestigungselements 100 und von mit dem Befestigungselement 100 verbundenen weiteren Teilen eines Implantats verbessert werden. Ein unbeabsichtigtes Verrutschen oder Herausrutschen des Befestigungselements 100 aus der Lamina kann so besonders gut vermieden werden.

Die vorliegende Erfindung ist jedoch nicht auf Ausführungsformen beschränkt, in denen das Halteelement 124 vorhanden ist. In anderen Ausführungsformen kann das Haltelement 124 weggelassen werden. In manchen dieser Ausführungsformen kann auch die Öffnung 130 der Platte weggelassen werden. In anderen Ausführungsformen kann die Öffnung 130 jedoch vorhanden sein, um je nach Bedarf das Anbringen des Halteelements 124 zu ermöglichen.

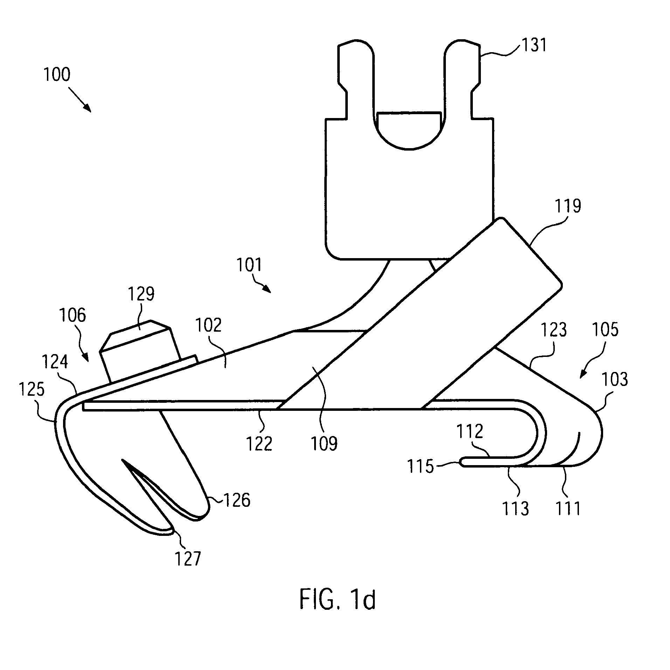

Wie in der Seitenansicht der Fig. 1d gezeigt, kann die Auflagefläche 122 des Auflageabschnitts 102 eine konkave Form haben. Dadurch kann eine Anpassung der Form der Auflagefläche 122 an die anatomische Form der Lamina erzielt werden, die eine Vergrößerung der Kontaktfläche des Befestigungselements 100 mit der Lamina ermöglicht.

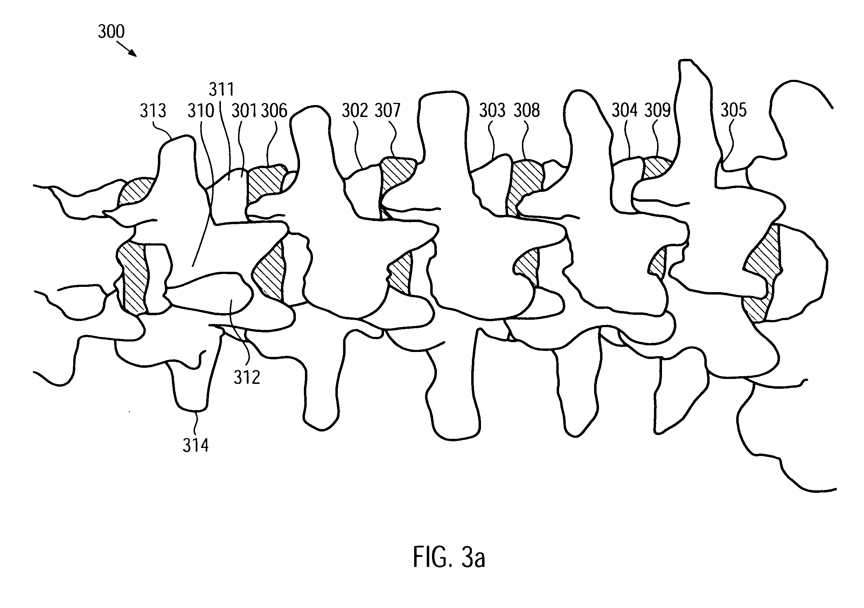

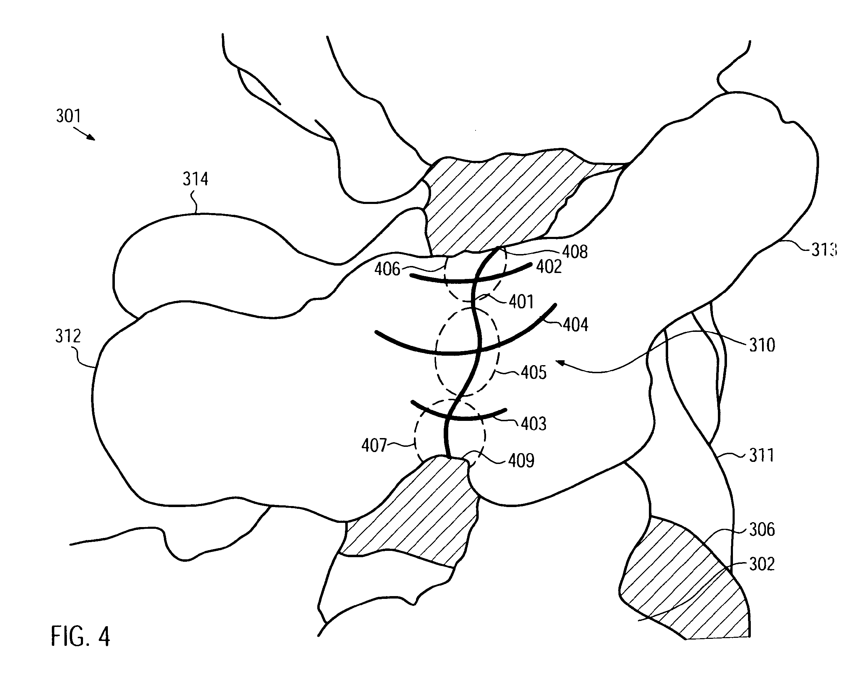

Die Form des Teils der Lamina, auf der die Auflagefläche 122 aufliegt, wird im Folgenden mit Bezug auf Fig. 4 erläutert. Fig. 4 zeigt eine schematische Perspektivansicht eines Wirbels 301. In den Fig. 3a bis 3d ist der Wirbel 301 aus einer anderen Perspektive sichtbar. Zwischen dem Wirbel 301 und einem benachbarten Wirbel 302 befindet sich eine Bandscheibe. Zwischen dem Dornfortsatz 312 und einem der beiden Querfortsätze 313 des Wirbels 301 ist die Lamina 310 zu erkennen. Die caudale Seite des Wirbels 301 ist in der Fig. 4 unten angeordnet, die craniale Seite des Wirbels 301 oben. Die Krümmung der Oberfläche der Lamina 310 wird schematisch durch Kurven 401, 402, 403, 404 dargestellt, die jeweils auf der Oberfläche der Lamina 301 verlaufen und in einer Ebene, die den Wirbel 301 schneidet, liegen.

Die Lamina 310 weist in der Nähe der cranialen Kante 408 einen Bereich 406 auf, in dem die Oberfläche der Lamina 310 eine sattelförmige Gestalt hat. Wie an dem Verlauf der Kurven 401 und 402 im Bereich 406 zu erkennen, ist die Oberfläche der Lamina 310 in der Richtung, in der die Linie 401 verläuft, vom Betrachter weg gekrümmt, während sie in der Richtung, in der die Linie 402 verläuft, auf den Betrachter zu gekrümmt ist.

In der Nähe der caudalen Kante 409 weist die Lamina 310 einen weiteren Bereich 407 auf, in dem ihre Oberfläche eine sattelförmige Gestalt hat, was am Verlauf der Kurven 401 und 403 in diesem Bereicht zu erkennen ist.

Zwischen den Bereichen 406, 407 befindet sich ein Bereich 405, in dem die Oberfläche der Lamina 310 eine muldenförmige Gestalt hat. Im Bereich 405 ist sowohl die Kurve 401 als auch die Kurve 404 auf den Betrachter zu gekrümmt.

Durch die konkave Form der Auflagefläche 122 kann erreicht werden, dass die Auflagefläche 122 sowohl im Bereich der Mulde 405 als auch in den sattelförmigen Bereichen 406, 407 auf der Oberfläche der Lamina 310 aufliegt, wodurch die Kontaktfläche zwischen dem Befestigungselement 100 und der Lamina 310 vergrößert werden kann.

Im Folgenden wird die Gestalt, die das Befestigungselement 100 in einer Ausführungsform der Erfindung haben kann, mit Bezug auf die Figuren 2a und 2b genauer erläutert. Die Figuren 2a und 2b zeigen die Platte 101, 501, wobei die Platte 101, 501 in der Ansicht der Fig. 2a aus der gleichen Richtung dargestellt ist wie in der Fig. 1b und in der Ansicht der Fig. 2b aus der gleichen Richtung dargestellt ist wie in der Fig. 1d. Zur besseren Darstellung ist die Platte 101, 501 in den Fig. 2a und 2b in einem geradegebogenen Zustand gezeigt. Die in den Fig. 1a, 1b und 1d gezeigte Form der Platte 101 kann aus der in den Fig. 2a und 2b gezeigten Form durch Biegen des gekrümmten Abschnitts 103 um die Krümmungsachse 117 sowie durch Biegen des Fortsatzes 119 in der in Fig. 1d und Fig. 2b nach oben weisenden Richtung erhalten werden. Die in Fig. 5a, 5b gezeigte Form der Patte 501 kann erhalten werden, indem der gekrümmte Abschnitt 503 zusätzlich derart verformt wird, dass er auf der von der Seite 106 abgewandten Seite eine "Einbuchtung" entsprechend der antiklastischen Form erhält. Dies kann beispielsweise durch Heißformen geschehen. Alternativ kann die antiklastische Form des gekrümmten Abschnitts auch durch Stanzen erzeugt werden.

Wie bereits oben erwähnt, kann die Platte 101, 501 in manchen Ausführungsformen eine Breite 108 im Bereich von 7 bis 13 mm haben. Eine Breite in diesem Bereich kann dem Abstand zwischen den Ansätzen des Dornfortsatzes 312 und des Gelenkfortsatzes 314 entsprechen, so dass das Befestigungselement 100 zwischen dem Gelenkfortsatz 314 und dem Dornfortsatz 312 eingesetzt werden kann.

Die Breite 108 kann an die individuelle Anatomie des Patienten und an den Wirbel, an dem das Befestigungselement 100 angebracht werden soll, angepasst sein. Da die Größe der Wirbel in der cranialen Richtung abnimmt, kann das Befestigungselement 100 eine größere Breite 108 aufweisen, wenn es an einem Lendenwirbel oder einem weiter unten gelegenen Brustwirbel angebracht werden soll, als wenn es an einem Wirbel im oberen Brustbereich oder einem Halswirbel angebracht werden soll.

Die längere der beiden Längsseiten 109, 110, die in der in Fig. 2a dargestellten Ausführungsform die Längsseite 110 ist, weist eine Länge 210 auf, wobei die Länge 210 von der dem gekrümmten Abschnitt 103 abgewandten Seite 106 bis zum Beginn des gekrümmten Abschnitts 103 gemessen werden kann. In manchen Ausführungsformen kann die Länge 210 im Bereich von 10 bis 25 mm liegen, beispielsweise ungefähr 22 oder ungefähr 23 mm betragen.

Der gekrümmte Abschnitt 103, 503 hat eine Länge 202, die vom Beginn des gekrümmten Abschnitts 103 auf der Seite des Auflageabschnitts 102 bis zum Ansatz der Vorsprünge 111, 112 gemessen werden kann. In manchen Ausführungsformen kann die Länge 202 im Bereich von 3 mm bis 7 mm liegen, beispielsweise ungefähr 5 mm betragen.

Der oben mit Bezug auf Fig. 5a, 5b beschriebene Oberflächenabschnitt 520 kann 50% oder mehr der Länge des gekrümmten Abschnitts 503 einnehmen. Im Rest des gekrümmten Abschnitts kann sich ein Übergangsbereich befinden, in dem die antiklastische Form in eine andere, beispielsweise zylindrische, gekrümmte Form übergeht. In manchen Ausführungsformen kann der Oberflächenabschnitt 520 aber auch die gesamte Länge des gekrümmten Abschnitts 503 einnehmen.

Der gekrümmte Abschnitt muss nicht, wie in Fig. 2a gezeigt, von zwei im Wesentlichen geraden Linien begrenzt sein. In manchen Ausführungsformen, in denen der gekrümmte Abschnitt, der in diesen Ausführungsformen durch das Bezugszeichen 503 bezeichnet ist, mit einer antiklastischen Form versehen wird, kann eine den beiden Vorsprüngen 111, 112 zugewandte Begrenzungslinie 550, wie in Fig. 5c dargestellt, derart gekrümmt sein, dass der gekrümmte Abschnitt in der Mitte zwischen den Längsseiten 109, 110 die oben beschriebene Länge 202 aufweist, an den Längsseiten 109, 110 jedoch eine größere Länge aufweist. In anderen Ausführungsformen kann jedoch auch im Fall eines antiklastisch gekrümmten Abschnitts 503 der gekrümmte Abschnitt 503 durch zwei gerade Linien begrenzt sein, wie in Fig. 2a gezeigt.

Die Vorsprünge 111, 112 haben eine Länge 203, die vom Ansatz des jeweiligen Vorsprungs 111, 112 bis zur Spitze des Vorsprungs 111, 112 gemessen werden kann. Die Länge beider Vorsprünge 111, 112 kann dabei gleich sein. In manchen Ausführungsformen kann die Länge 203 der Vorsprünge 111, 112 im Bereich von 3 mm bis 10 mm liegen, beispielsweise ungefähr 5 mm oder ungefähr 8 mm betragen.

Eine größere Länge 203 der Vorsprünge 111, 112 kann eine stabilere Befestigung des Befestigungselements 100 an der Lamina ermöglichen. Da sich die Vorsprünge 111, 112 beim implantierten Befestigungselement 100 jedoch auf der dem Rückenmark des Patienten zugewandten Seite der Lamina 310 befinden, sollte die Länge 203 derart angepasst sein, dass ein Sicherheitsabstand zum Rückenmark eingehalten wird, um Verletzungen des Rückenmarks zu vermeiden. Da sich die craniale (obere) Kante 408 der Lamina 310 näher am Rückenmark befindet als die caudale (untere) Kante 409, können die Vorsprünge 111, 112 eine größere Länge aufweisen, wenn das Befestigungselement 100 zum Einhängen des gekrümmten Abschnitts 103, 503 in die caudale Kante 409 vorgesehen ist, als wenn das Befestigungselement 100 zum Einhängen des gekrümmten Abschnitts 103 in die craniale Kante 408 vorgesehen ist. Beispielsweise kann die Länge 203, wenn der gekrümmte Abschnitt 103 in die craniale Kante 408 eingehängt werden soll, ungefähr 5 mm betragen, und die Länge 203 kann ungefähr 8 mm betragen, wenn der gekrümmte Abschnitt 103 in die caudale Kante 409 eingehängt werden soll.

In den vom gekrümmten Abschnitt 103, 503 abgewandten Teilen 113, 115 können die Vorsprünge 111, 112 mit abgestumpften Kanten, beispielsweise mit abgerundeten Kanten, ausgebildet werden. Dadurch kann das Risiko, beim Einsetzen des Befestigungselements 100 das Rückenmark des Patienten zu verletzen, weiter verringert werden.

Der Fortsatz 119 kann eine Länge 204 im Bereich von 17 bis 25 mm, beispielsweise eine Länge von ungefähr 21 mm oder ungefähr 20 mm und eine Breite 205 im Bereich von 4 mm bis 8 mm, beispielsweise eine Breite von ungefähr 6 mm haben. Ein Abstand 206 zwischen der dem gekrümmten Bereich 103, 503 zugewandten Seite des Fortsatzes 119 und dem gekrümmten Abschnitt 103 kann im Bereich von ungefähr 1 mm bis ungefähr 3 mm liegen, beispielsweise ungefähr 2 mm betragen.

Die Form der Auflagefläche 122 und der Deckfläche 123 in einer Ausführungsform des Befestigungselements 100 gemäß der vorliegenden Erfindung wird im Folgenden mit Bezug auf Fig. 2b beschrieben.

In Fig. 2b bezeichnet das Bezugszeichen 255 eine Ebene, die durch Bereiche der Auflagefläche 122 neben dem gekrümmten Abschnitt 103, 503 und durch Bereiche der Auflagefläche 122 auf der Seite 106 der Platte 101, 501 die dem gekrümmten Abschnitt 103 gegenüberliegt, verläuft. Insbesondere kann die Ebene 255 durch den Rand der Auflagefläche 122 auf der Seite 106 und durch einen Punkt der Auflagefläche unmittelbar neben dem gekrümmten Abschnitt 103, 503 verlaufen.

Aufgrund der konvexen Form der Auflagefläche 122 liegen Teile der Auflagefläche nicht in der Ebene 255, sondern befinden sich in einem Abstand zu der Ebene. Der Abstand eines Punkts auf der Auflagefläche 122 von der Ebene kann dabei in einer zu der Ebene 255 senkrechten Richtung gemessen werden. Die Auflagefläche 122 umfasst einen oder mehrere Punkte, die von der Ebene 255 einen Abstand aufweisen, der größer oder gleich dem Abstand aller anderen Punkte der Auflagefläche 122 von der Ebene 255 ist. Der Abstand dieser Punkte von der Ebene 255 wird im Folgenden als "maximaler Abstand" bezeichnet, und die Punkte, die sich in dem maximalen Abstand von der Ebene 255 befinden, werden als "Punkte maximalen Abstands" bezeichnet.

In Fig. 2b bezeichnet das Bezugszeichen 250 einen im Wesentlichen flachen Teil der Auflagefläche 122, der im Wesentlichen parallel zu der Ebene 255 ist. In manchen Ausführungsformen kann der Teil 255 kreisförmig mit einem Durchmesser 254 sein. Der Durchmesser 254 kann einen Wert im Berech von 3 mm bis 6 mm, beispielsweise einen Wert von ungefähr 4 mm, haben. Punkte auf der Auflagefläche im Teil 250 befinden sich in einem Abstand 257 von der Ebene 255.

Der flache Teil 250 der Auflagefläche 122 kann von einem Teil 251 mit einer im Wesentlichen konischen Form umgeben sein. Punkte in dem konischen Teil 251 der Auflagefläche 122 befinden sich in einem Abstand zu der Ebene 255, der kleiner als der Abstand der Punkte im flachen Teil 250 der Auflagefläche 122 von der Ebene 255 ist. Somit bildet der flache Teil 250 der Auflagefläche 122 eine Menge von Punkten maximalen Abstands von der Ebene 255.

Außerhalb einer Schnittlinie des konischen Teils 251 mit der Ebene 255 kann die Auflagefläche 122 im Wesentlichen in der Ebene 255 liegen, so dass die Auflagefläche in der Umgebung der durch die Seiten 105, 106 und die Längsseiten 109, 110 gebildeten Ecken im Wesentlichen flach ist. In der Mitte der Seiten 105, 106 kann sich der konische Bereich 122 bis zum Rand des Auflageabschnitts 102 erstrecken und dort die Ebene 255 schneiden.

Der konische Teil 251 der Auflagefläche 122 muss sich nicht, wie in Fig. 2b gezeigt, vom Rand des flachen Teils 250 bis zum Rand des Auflageabschnitts 102 auf den Seiten 105, 106 der Platte 101, 501 erstrecken. In manchen Ausführungsformen kann der konische Teil 251 den flachen Teil 250 ringförmig umschließen. Außerhalb des konischen Teils 251 kann die Auflagefläche 122 im wesentlichen in der Ebene 255 liegen.

Wenn das Befestigungselement 100 an der Lamina angebracht wird, können der flache Teil 250 und der konische Teil 251 zumindest teilweise in dem muldenförmigen Bereich 405 der Lamina 310 aufliegen. Dadurch kann die Kontaktfläche zwischen der Lamina 310 und der Platte 101 vergrößert werden. Die zumindest teilweise Auflage des flachen Teils 250 und/oder des konischen Teils kann dabei bei gleichbleibenden Abmessungen in einem gewissen Bereich von Dimensionen des Wirbels erreicht werden, so dass gleiche Befestigungselemente 100 auf unterschiedlich großen Wirbeln verwendet werden können. Es ist deshalb möglich, eine Vielzahl unterschiedlich dimensionierter Wirbel mit einer relativ geringen Auswahl an unterschiedlich dimensionierten Befestigungselementen zu versorgen, wodurch eine wirtschaftliche Produktion der Befestigungselemente ermöglicht wird.

Die vorliegende Erfindung ist nicht auf Ausführungsformen beschränkt, in der die Auflagefläche 122 die oben beschriebene Form hat. In anderen Ausführungsformen kann die Auflagefläche 122 zumindest teilweise eine sphärische, hyperboloide, paraboloide oder ellipsoide Form haben.

Der Abstand 257 der Punkte maximalen Abstands von der Ebene 255 kann einen Wert im Bereich von 1 mm bis 2 mm, beispielsweise eine Wert von ungefähr 1,5 mm haben.

Ein Abstand zwischen dem Mittelpunkt des flachen Teils 250 und der Seite 106 kann einen Wert im Bereich von ungefähr 7 bis ungefähr 11 mm haben, beispielsweise einen Wert von ungefähr 8 mm oder ungefähr 10 mm.

Das Verbindungselement 131 kann gegenüber von mindestens einem der Punkte maximalen Abstands von der Ebene 255 angeordnet sein. Beispielsweise kann in Ausführungsformen, in denen die Platte 101 die in Fig. 2b dargestellte Form hat, das Haltelement 130 gegenüber dem flachen Teil 250 der Auflagefläche 122 angeordnet sein.

Ähnlich wie die Auflagefläche 122 kann auch die Deckfläche 123 eine konvexe Form haben. Die Deckfläche 123 kann einen oder mehrere Punkte maximalen Abstands zu einer Ebene 256 aufweisen, die durch Bereiche der Deckfläche 123 neben dem gekrümmten Abschnitt 103 und durch Bereiche der Deckfläche 123 auf der Seite 106, die dem gekrümmten Abschnitt 103, 503 gegenüberliegt, verläuft. Die Punkte maximalen Abstands können von der Ebene 256 einen Abstand 258 im Bereich von 3 bis 5 mm, beispielsweise einen Abstand von ungefähr 4 mm haben. In manchen Ausführungsformen kann die Deckfläche 123 einen im wesentlichen flachen Teil 252, der parallel zu der Ebene 256 ist und sich im Abstand 258 von dieser befindet, sowie einen im wesentlichen kegelförmigen Teil 253, der den Teil 252 umgibt, aufweisen. Der flache Teil 252 der Deckfläche 123 kann sich gegenüber dem flachen Teil 250 der Auflagefläche 122 befinden. Der Durchmesser der flachen Teile 250, 252 kann ungefähr gleich sein. Das Haltelement 130 kann in dem flachen Teil 252 angeordnet sein, so dass es mit einem Bereich maximaler Dicke der Platte verbunden ist. Dadurch kann die Steifigkeit des Befestigungselements 100 verbessert werden.

Ein Implantat zur Korrektur einer Wirbelsäulendeformität gemäß der vorliegenden Erfindung kann zwei oder mehr Befestigungselemente mit den Merkmalen des oben beschriebenen Befestigungselements 100 umfassen. Die Form der Befestigungselemente kann dabei je nach der Art der Anbringung des Implantats an der Wirbelsäule des Patienten angepasst werden.

Die Form des Befestigungselements 100 kann sich unterscheiden, je nachdem, ob es zum Einhängen des gekrümmten Abschnitts 103, 503 in die craniale (obere) Kante 408 der Lamina 310 oder zum Einhängen des gekrümmten Abschnitts 103, 503 in die caudale (untere) Kante 409 der Lamina 310 ausgelegt ist.

Wie bereits oben ausgeführt, kann das Befestigungselement für die caudale Kante eine größere Breite 108 als das Befestigungselement für die craniale Kante aufweisen. In einer speziellen Ausführungsform kann das Befestigungselement für die caudale Kante eine Breite von 10 oder 12 mm haben und das Befestigungselement für die crainale Kante kann eine Breite von 8 oder 10 mm haben. Außerdem können, wie oben ausgeführt, die Vorsprünge 111, 112 des Befestigungselements für die caudale Kante eine größere Länge aufweisen.

Das Befestigungselement für die craniale Kante kann mit dem Fortsatz 119 zum Einhängen in den Dornfortsatz des Wirbels des Patienten ausgebildet sein, während bei dem Befestigungselement für die caudale Kante der Fortsatz 119 weggelassen werden kann.

Ferner können unterschiedliche Befestigungselemente zum Anbringen auf der linken oder der rechten Lamina bereitgestellt werden, wobei die Befestigungselemente für die linke Seite spiegelsymmetrisch zu denen für die rechte Seite sind.

Bei der Implantation des Befestigungselements 100 werden die kürzere Längsseite 109 und der Fortsatz 119 neben dem Dornfortsatz des Wirbels angebracht. Die längere Seite 110 befindet sich dann neben einem der Querfortsätze des Wirbels.

Die Implantation eines Implantats mit zwei Befestigungselementen gemäß der vorliegenden Erfindung wird im Folgenden mit Bezug auf die Fig. 3a bis 3d beschrieben.

Fig. 3a zeigt eine schematische Darstellung eines Teils einer Wirbelsäule 300 eines Patienten. In Fig. 3a zeigt die caudale Richtung nach rechts und die craniale Richtung zeigt nach links. Die Wirbelsäule 300 umfasst Wirbel 301, 302, 303, 304, 305, zwischen denen sich Bandscheiben 306, 307, 308, 309 befinden.

Der Wirbel 301 umfasst einen Wirbelkörper 311, einen Dornfortsatz 312 und Querfortsätze 313, 314. Zwischen dem Querfortsatz 313 und dem Dornfortsatz 312 befindet sich eine Lamina 310. Eine weitere Lamina befindet sich zwischen dem Dornfortsatz 312 und dem Querfortsatz 314. Die Form der Lamina 310 wurde oben mit Bezug auf Fig. 4 genauer erläutert. Die Wirbel 302 bis 305 haben eine ähnliche Form wie der Wirbel 301, sind jedoch etwas größer, da sie weiter caudal liegen.

Vor der Implantation wird der Teil der Wirbelsäule 300, der mit Hilfe des Implantats korrigiert und/oder stabilisiert werden soll, zum Beispiel die Wirbel 301 bis 304, freigelegt. Dies kann mit Hilfe den Fachleuten bekannter Operationstechniken geschehen.

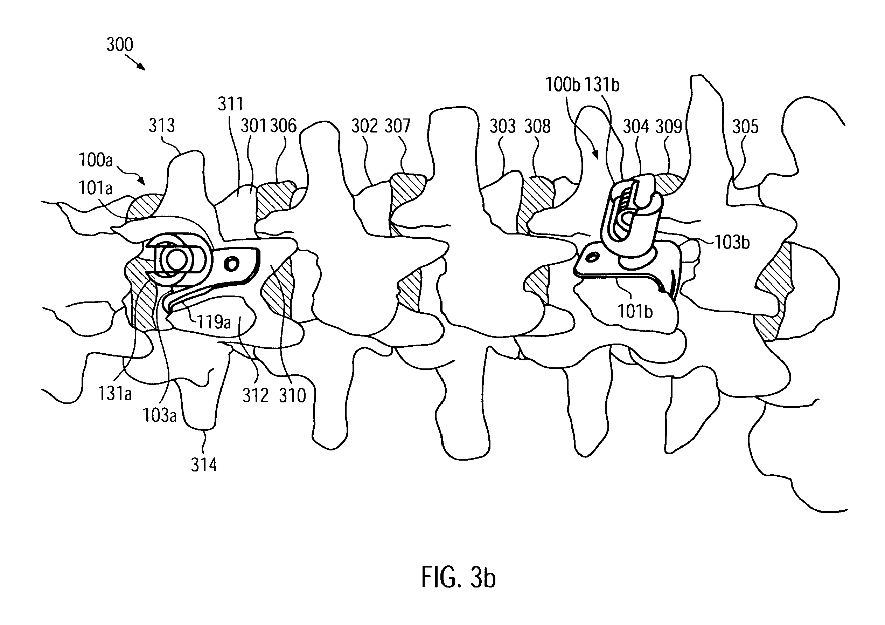

Fig. 3b zeigt den in Fig. 3a gezeigten Teil der Wirbelsäule 300 in einem späteren Stadium der Implantation.

An der Lamina 310 des Wirbels 301 ist eine Platte 101a eines ersten Befestigungselements 100a gemäß der vorliegenden Erfindung angebracht, die mit einem ersten Verbindungselement 131a verbunden ist. An der Lamina des Wirbels 304 ist die Platte 101b eines zweiten Befestigungselements 100b gemäß der vorliegenden Erfindung angebracht, die mit einem zweiten Verbindungselement 131b verbunden ist. Die Befestigungselemente 100a, 100b können Merkmale entsprechend denen, die oben mit Bezug auf die Fig. 1a bis 1d, 2a, 2b und 5a, 5b beschrieben wurden, aufweisen. Im Folgenden werden zur Bezeichnung von Teilen des ersten Befestigungselements 100a die in den Fig. 1a bis 1d sowie 2a und 2b verwendeten Bezugszeichen mit nachgestelltem "a" verwendet, während zur Bezeichnung von Teilen des zweiten Befestigungselements 100b Bezugszeichen mit nachgestelltem "b" verwendet werden. Die Platten 101a, 101b können die Merkmale der oben mit Bezug auf Fig. 1a bis 1d beschriebenen Platte 101, oder die Merkmale der oben mit Bezug auf Fig. 5a, 5b beschriebenen Platte 501 aufweisen. Die mit den Bezugszeichen 103a, 103b gekennzeichneten gekrümmten Abschnitte können zylindrisch gekrümmte Abschnitte oder antiklastisch gekrümmte Abschnitte sein.

Der gekrümmte Abschnitt 103a der Platte 101a des ersten Befestigungselements 100a wird in die craniale Kante der Lamina 310 des Wirbels 301 eingehängt und der gekrümmte Abschnitt 103b der Platte 101b des zweiten Befestigungselements 100b wird in die caudale Kante der Lamina des Wirbels 304 eingehängt. Ein Durchtrennen des gelben Bandes ist dabei nicht erforderlich. Stattdessen können die Vorsprünge der Befestigungselemente 100a, 100b (in Fig. 3b nicht sichtbar) in das gelbe Band eingeklemmt werden. Die gekrümmten Abschnitte 103a, 103b können zwischen der Basis der Dornfortsätze der Wirbel 301, 304 und der medialen Kante der Gelenkfortsätze der Wirbel 302, 304 eingeklemmt werden.

Das erste Befestigungselement 100a weist einen Fortsatz 119a auf, der in den Dornfortsatz 312 des Wirbels 301 eingehängt wird. Wie bereits oben erwähnt, kann der Fortsatz 119a zunächst im wesentlichen gerade sein (wie in Fig. 1a bis 2b gezeigt) und während der Operation durch Biegen mit Hilfe einer Zange in eine hakenförmige Form gebracht werden. Dadurch kann eine Anpassung der Form des Fortsatzes 119 an die individuelle Anatomie des Patienten erreicht werden. Das zweite Befestigungselement 100b umfasst in dieser Ausfübrungsform keinen Fortsatz.

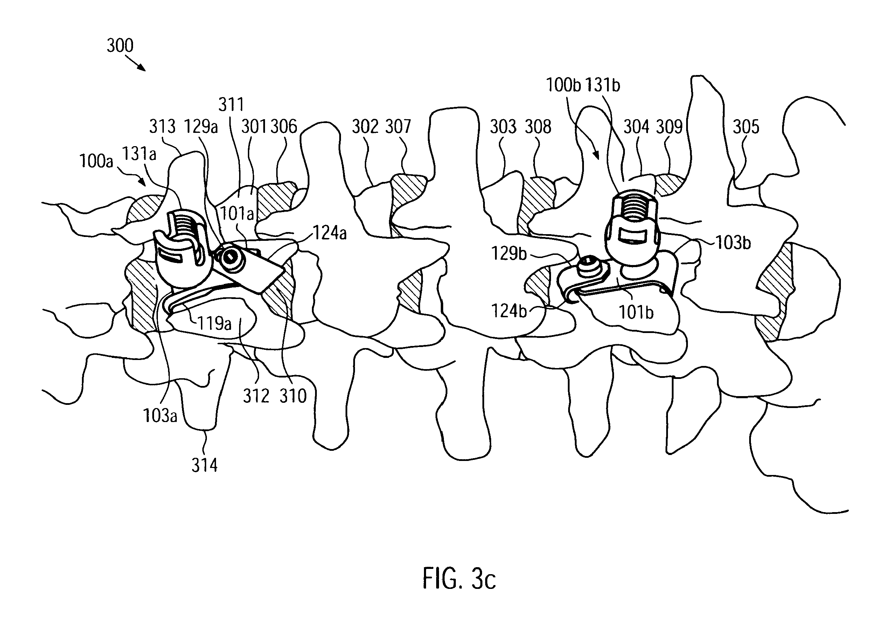

Fig. 3c zeigt die Wirbelsäule 300 in einem späteren Stadium der Implantation. Ein Halteelement 124a des ersten Befestigungselements wird in die caudale Kante der Lamina 310 des Wirbels 301 eingehängt und durch eine Schraube 129a mit der Platte 101a des ersten Befestigungselements 100a verbunden. Entsprechend wird ein Halteelement 124b in die craniale Kante der Lamina des Wirbels 304 eingehängt und mit Hilfe einer Schraube 129b mit der Platte 101b des zweiten Befestigungselements verbunden.

In anderen Ausführungsformen der Erfindung können eines oder beide der Halteelemente 124a, 124b weggelassen werden. Die Halteelemente 124a, 124b können auch je nach Bedarf verwendet werden, wenn der Grad an primärer Stabilität, der ohne die Haltelemente 124a, 124b erreicht wird, als unzureichend angesehen wird. Wenn die Platten 101a, 101b auch ohne die Haltelemente 124a, 124b eine ausreichende primäre Stabilität aufweisen, können die Halteelemente 124a, 124b dagegen weggelassen werden. Die korrigierende und stabilisierende Funktion der Befestigungselemente 100a, 100b kann auch ohne die Halteplatten 124a, 124b erreicht werden, während die Halteelemente 124a, 124b als Hilfsmittel zur intraoperativen Manipulation verwendet werden können.

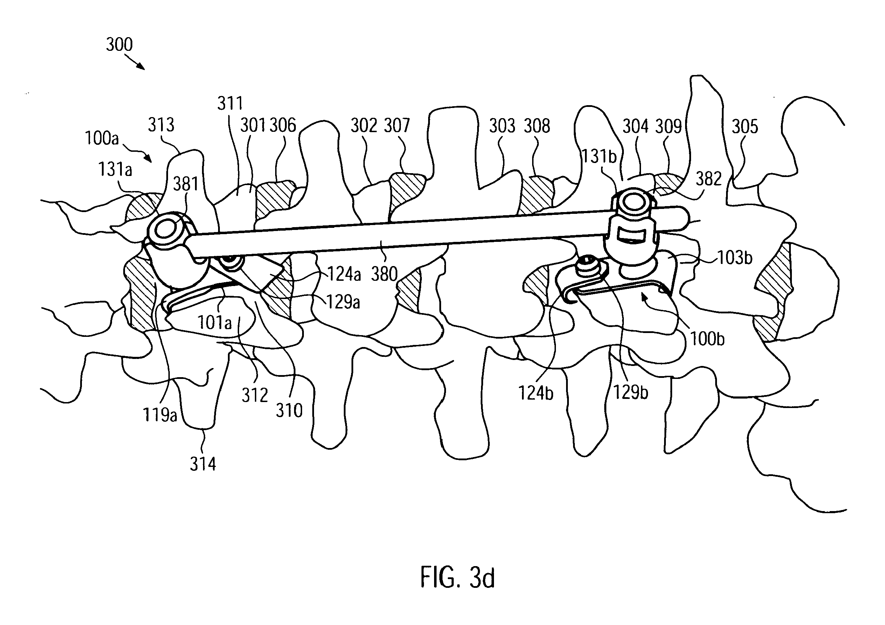

Fig. 3d zeigt die Wirbelsäule 300 in einem weiteren Stadium der Implantation. Nachdem die Befestigungselemente 100 an den Wirbeln 301, 304 angebracht sind, wird die Wirbelsäule 300 durch Bewegen des Patienten in einer gewünschten Stellung ausgerichtet. Anschließend kann der Abschnitt der Wirbelsäule 300 zwischen den Wirbeln 301 und 304 durch Einsetzen eines Verbindungsstabes 380 in die Verbindungselemente 130a, 130b und Fixieren des Verbindungsstabes 380 in den Verbindungselementen 130a, 130b, beispielsweise mit Hilfe von Schrauben 381, 382, die in die Gewinde der Verbindungselemente 130a, 130b eingeschraubt werden, in der gewünschten Stellung stabilisiert und fixiert.

In manchen Ausführungsformen können die Haltelemente 124a, 124b nach dem Fixieren des Verbindungsstabs 380 entfernt werden. In anderen Ausführungsformen können sie dagegen im Körper des Patienten verbleiben.

Anschließend können, je nach Bedarf, weitere, den Fachleuten bekannte, Operationsschritte durchgeführt werden, und der Zugang zu der Wirbelsäule 300 kann verschlossen werden.

Bei geriatrischen Patienten kann eine Indikation zur Anwendung eines Implantats gemäß der vorliegenden Erfindung im korrigierenden und stabilisierenden Verfahren bestehen, wenn ein Schmerzsyndrom mit oder ohne neurologischen Ausfall trotz einer drei- bis sechsmonatigen konservativen Behandlung anhält und beispielsweise eine der folgenden Diagnosen vorliegt:
1. degenerative Skoliose;
2. posttraumatische Deformität, z.B. eine posttraumatische Kyphose nach einer Serienfraktur; oder
3. mehrsegmentale Knochenmetastasen.

## Patentansprüche

1. Befestigungselement zum Befestigen eines Implantats zur Korrektur einer Wirbelsäulendeformität an einer Lamina eines Wirbels, umfassend
eine Platte mit einem Auflageabschnitt zur Auflage auf der Lamina, wobei die Platte an einer Seite einen gekrümmten Abschnitt zum Einhängen der Platte in die Lamina aufweist, wobei eine Längsrichtung der Platte von der Seite mit dem gekrümmten Abschnitt zu einer der Seite mit dem gekrümmten Abschnitt gegenüberliegenden Seite verläuft, eine Querrichtung der Platte senkrecht zur Längsrichtung verläuft und die Platte in der Querrichtung eine Breite von 7 mm bis 13 mm aufweist.

2. Befestigungselement nach Anspruch 1, das zwei Längsseiten aufweist, die parallel zu der Längsrichtung der Platte sind.

3. Befestigungselement nach einem der Ansprüche 1 und 2, das zwei oder mehr Vorsprünge aufweist, die sich an den gekrümmten Abschnitt anschließen, wobei jeder Vorsprung einen vom gekrümmten Abschnitt der Platte abgewandten Teil aufweist, der gegenüber dem Auflageabschnitt der Platte angeordnet ist.

4. Befestigungselement nach einem der Ansprüche 1 bis 3, wobei der gekrümmte Abschnitt einen Oberflächenabschnitt aufweist, welcher der dem gekrümmten Abschnitt gegenüberliegenden Seite der Platte zugewandt ist, wobei für jeden Punkt in dem Oberflächenabschnitt gilt, dass ein Hauptnormalschnitt des Oberflächenabschnitts in dem Punkt in einer Ebene liegt, deren Normale schräg zu der Längrichtung ist.

5. Befestigungselement nach Anspruch 4, wobei der gekrümmte Abschnitt eine zylindrische Krümmung aufweist, wobei eine Krümmungsachse des gekrümmten Abschnitts der Platte schräg zu der Längsrichtung verläuft.

6. Befestigungselement nach Anspruch 5, wobei die Krümmungsachse mit der Längsrichtung einen Winkel im Bereich von 95 bis 115 Grad einschließt.

7. Befestigungselement nach Anspruch 4, wobei der Oberflächenabschnitt eine antiklastische Fläche ist, und der Mittelpunkt des zu dem genannten Hauptnormalschnitt gehörigen Krümmungskreises auf der Seite des gekrümmten Abschnitts liegt, die der dem gekrümmten Abschnitt gegenüberliegenden Seite der Platte zugewandt ist.

8. Befestigungselement nach einem der Ansprüche 2, 3, 4 und 5, wobei der Auflageabschnitt an einer der beiden Längsseiten einen Fortsatz, insbesondere einen verformbar ausgebildeten Fortsatz, aufweist, der sich in einer zur Längsrichtung der Platte schrägen Richtung erstreckt.

9. Befestigungselement nach einem der Ansprüche 1 bis 10, wobei der Auflageabschnitt eine konvexe Auflagefläche, insbesondere eine Auflagefläche, von der zumindest ein Teil eine konische Form hat, aufweist und wobei der gekrümmte Abschnitt zu der Auflagefläche hin gekrümmt ist.

10. Befestigungselement nach Anspruch 9, wobei die Auflagefläche mindestens einen Punkt maximalen Abstands zu einer Ebene aufweist, die durch Bereiche der Auflagefläche neben dem gekrümmten Abschnitt und durch Bereiche der Auflagefläche auf der Seite der Platte, die der Seite mit dem gekrümmten Abschnitt gegenüberliegt, verläuft, und der mindestens eine Punkt maximalen Abstands von der Ebene einen Abstand im Bereich von 1 bis 2 mm hat.

11. Befestigungselement nach einem der Ansprüche 1 bis 10, wobei der Auflageabschnitt eine konvexe Deckfläche, insbesondere eine Deckfläche, von der zumindest ein Teil eine konische Form hat, aufweist und wobei der gekrümmte Abschnitt von der Deckfläche weg gekrümmt ist.

12. Befestigungselement nach Anspruch 11 wobei die Deckfläche mindestens einen Punkt maximalen Abstands zu einer Ebene aufweist, die durch Bereiche der Deckfläche neben dem gekrümmten Abschnitt und durch Bereiche der Deckfläche auf der Seite der Platte, die der Seite mit dem gekrümmten Abschnitt gegenüberliegt, verläuft, und der mindestens eine Punkt maximalen Abstands von der Deckfläche einen Abstand im Bereich von 3 bis 5 mm hat.

13. Befestigungselement nach einem der Ansprüche 1 bis 12, das ferner umfasst:
ein Halteelement, das einen gekrümmten Abschnitt zum Einhängen des Haltelements in die Lamina aufweist, und das derart an dem Befestigungselement anbringbar ist, dass der gekrümmte Abschnitt des Haltelements dem gekrümmten Abschnitt des Befestigungselements gegenüber angeordnet ist.

14. Implantat zur Korrektur einer Wirbelsäulendeformität, umfassend:
ein erstes Befestigungselement nach einem der Ansprüche 1 bis 13;
ein zweites Befestigungselement nach einem der Ansprüche 1 bis 13;
einen Verbindungsstab; und
Mittel zum Befestigen des Verbindungsstabs an dem ersten Befestigungselement und dem zweiten Befestigungselement.

15. Implantat nach Anspruch 14,
wobei das erste und das zweite Befestigungselement jeweils zwei Vorsprünge umfassen, die sich an den gekrümmten Abschnitt des Befestigungselements anschließen und die Vorsprünge des zweiten Befestigungselements länger als die Vorsprünge des ersten Befestigungselements sind.

16. Implantat nach einem der Ansprüche 14 und 15, wobei das zweite Befestigungselement breiter als das erste Befestigungselement ist.

17. Implantat nach einem der Ansprüche 14 bis 16, wobei das erste Halteelement einen Fortsatz aufweist, der sich in einer zur Längsrichtung der Platte schrägen Richtung erstreckt.
